Europäisches Patentamt

European Patent Office

Office européen des brevets

(11) Publication number: **0 298 501 B1**

# EUROPEAN PATENT SPECIFICATION

(45) Date of publication of patent specification: **08.04.92**

(51) Int. Cl.⁵: **A61K 6/06**, A61L 27/00, A61K 6/08

(21) Application number: **88110950.8**

(22) Date of filing: **08.07.88**

Divisional application 91102716.7 filed on 08/07/88.

(54) **Composition for forming calcium phosphate type hardening material and process for producing such hardening material.**

(30) Priority: **10.07.87 JP 172445/87**
**10.07.87 JP 172446/87**
**13.10.87 JP 257939/87**
**13.10.87 JP 257940/87**
**10.06.88 JP 143122/88**

(43) Date of publication of application:
**11.01.89 Bulletin 89/02**

(45) Publication of the grant of the patent:
**08.04.92 Bulletin 92/15**

(84) Designated Contracting States:
**DE FR GB**

(56) References cited:
**WO-A-86/01113**
**US-A- 4 518 430**

**JAPANESE PATENTS GAZETTE, section Ch, week 8830, 7th September 1988, abstract no. 88-208078/30, Derwent Publications Ltd, London, GB; & JP-A-63 143 071 (M. NAGASE) 15-06-1988**

**PATENT ABSTRACTS OF JAPAN, vol. 11, no. 192 (C-429)[2639], 19th June 1987; & JP-A-62**

**12 705 (MEISHIN K.K.) 21-01-1987**

(73) Proprietor: **ASAHI KOGAKU KOGYO KABUSHIKI KAISHA**
**36-9, Maeno-cho 2-Chome Itabashi-ku Tokyo 174(JP)**

(72) Inventor: **Sumita, Masaya c/o Asahi Kogaku Kogyo K.K.**
**36-9 Maeno-cho 2-chome**
**Itabashi-ku Tokyo(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

The present invention relates to a composition for forming calcium phosphate type hardening material and a process for producing such a hardening material. More particularly, the present invention relates to a composition for forming a calcium phosphate type hardening material which is useful as a medical or dental cement material or a bone prosthetic material. The present invention also relates to a process for producing a hardening material in block form or other hardening materials useful as artificial bones and dental roots.

### BACKGROUND OF THE INVENTION

Calcium phosphate compounds, in particular hydroxyapatite, have an excellent biocompatibility and their use as biomaterials in medical or dental fields has been widely investigated.

Calcium phosphate compounds other than apatite are known to be converted to hydroxyapatite upon hydrolysis, and it has recently been found that the hydroxyapatite so produced can be hardened under certain conditions. In an attempt to exploit this phenomenon, several efforts have been made to use calcium phosphate powders as dental or medical cement materials (as described, for example, Japanese Patent Application (OPI) Nos. 12705/87, 161206/86, 182263/84 and 88351/84) (the term "OPI" as used herein means an unexamined published Japanese patent application)

Calcium phosphate type hardening materials are currently made from three types of calcium phosphate, i.e., a-tricalcium phosphate ($Ca_3(PO_4)_2$), tetracalcium phosphate ($Ca_4(PO_4)_2$),and mixtures of these two compounds. In order to prepare $\alpha$-tricalcium phosphate, calcium carbonate is mixed with calcium pyrophosphate and the mixture is calcined at 1,250°C. To prepare tetracalcium phosphate, calcium carbonate and calcium pyrophosphate are finely ground and mixed, followed by the mixture being calcined at 1,500°C. A mixture of $\alpha$-tricalcium phosphate and tetracalcium phosphate is prepared by finely grinding and mixing the respective ingredients prepared by the above-described methods.

Since these methods involve a solid phase reaction, the powders obtained lack uniformity in composition. With such powders, a hardening reaction therefore does not proceed uniformly and this results in a hardening material having a reduced strength. Furthermore, because of the high firing temperatures employed, the powders prepared by these conventional methods have low activity and suffer from such disadvantages as a prolonged hardening time and a decrease in the strength of the resulting hardening materials on account of incomplete hardening.

Calcium phosphate powders are hardened by mixing them with a hardening solution. In order to ensure that the hardening material will do no harm to body tissues, it is desirable for the mixture to have a low pH and that the hardening reaction will proceed in the neutral range. An aqueous solution of citric acid has heretofore been considered the best hardening solution capable of satisfying these requirements. However, such an aqueous solution of citric acid still leaves room for improvement, especially when compared with acrylic cement materials which have been commonly used in the art. The considerable problem is that a mixture of a calcium phosphate powder and an aqueous citric acid solution is not highly plastic and cannot be readily formed into a desired shape.

### SUMMARY OF THE INVENTION

An object of the invention is to provide an improved composition for forming a calcium phosphate type hardening material. This composition employs a powder component which can be produced by any suitable method, allows a hardening reaction to proceed uniformly and thoroughly in the neutral range, can be readily shaped into a desired form by kneading operations, and produces a hardening material which has high strength and yet does not harm body tissues.

Another object of the present invention is to provide a process for producing such a calcium phosphate type hardening material.

Other objects and effects of the present invention will be apparent from the following description.

As a result of various studies conducted in order to solve the aforementioned problems of the prior art, the present inventors have found that a mixture having good formability could be made from an $\alpha$-tricalcium phosphate and/or tetracalcium phosphate powder by using an aqueous acidic hardening solution containing a polysaccharide as a hardening solution. The present inventors also found that a hardened material of good quality could be produced from such a mixture. The present invention has been accomplished on the basis of these findings.

The present invention provides, in one aspect, a composition for forming a calcium phosphate type hardening material comprising (1) a powder comprising at least one of $\alpha$-tricalcium phosphate and tetracalcium phosphate, (2) an acidic aqueous solution, and (3) a polysaccharide which is preferably dissolved in the acidic aqueous solution.

In another aspect, the present invention provides a process for producing a calcium phosphate type hardening material by mixing the powder (1) with the aqueous acidic solution (2).

BRIEF DESCRIPTION OF THE DRAWINGS

Fig. 1 is a X-ray diffraction scan of the hydroxyapatite synthesized in Example 19; and
Fig. 2 is a X-ray diffraction scan of the powder prepared in Example 19 in accordance with the present inveniton.

DETAILED DESCRIPTION OF THE INVENTION

In the present invention, $\alpha$-tricalcium phosphate, tetracalcium phosphate or a mixture thereof is used as a powder component. Synthesis of $\alpha$-tricalcium phosphate can be made by a known dry or wet process. Tetracalcium phosphate can be prepared by a known dry process involving the reaction between calcium pyrophosphate and calcium carbonate. The powder components prepared need not be completely pure and they may contain minor amounts of impurities which are generated during the synthesis. If a mixture of $\alpha$-tricalcium phosphate and tetracalcium phosphate is used, their proportions are not limited to any particular values, and the two compounds may be synthesized separately and thereafter mixed in appropriate proportions.

A mixture of $\alpha$-tricalcium phosphate and tetracalcium phosphate which is preferably used in the present invention can be prepared by the step of calcining, at a temperature of from about 1,150°C to 1,450°C under reduced pressure, a hydroxyapatite having a molar ratio of Ca/P of about 1.8 or less and more than 1.5 so as to produce a mixture of $\alpha$-tricalcium phosphate and tetracalcium phosphate.

In this method for producing the mixture using a reduced pressure, the hydroxyapatite having a molar ratio of Ca/P of more than 1.5 but not exceeding about 1.8 can be readily synthesized by a wet method. If such hydroxyapatite is calcined under reduced pressure at about 1,150°C or more, it is thermally decomposed to form $\alpha$-tricalcium phosphate and tetracalcium phosphate in admixture. The resulting mixture is uniform in composition. Hydroxyapatite generally starts to be thermally decomposed at a temperature near 1,400°C if the pressure is atmospheric. In the method described above, the pressure is subatmospheric, so the reaction of thermal decomposition is allowed to proceed at a fairly high speed even at the comparatively low temperature of about 1,150°C. This is beneficial to the purpose of simplifying the manufacturing steps and reducing the production cost. The low calcining temperature offers the additional advantage of providing a highly active powder for use as a component in calcium phosphate type hardening materials.

The hydroxyapatite used as the starting material in the method described above can be readily synthesized by a wet process in which an aqueous solution of phosphoric acid is reacted with a suspension of calcium hydroxide by a known method. The molar ratio of Ca/P of this hydroxyapatite must be more than 1.5 and about 1.8 or less, preferably from about 1.6 to 1.8. If the molar ratio of Ca/P is 1.5, the product thus-obtained does not become a mixture but $\alpha$-tricalcium phosphate per se. If the molar ratio of Ca/P exceeds about 1.8, calcium oxide which is deleterious to the human body will form during calcining. The Ca/P molar ratio of the hydroxyapatite used as the starting material can be adjusted by changing the proportions of calcium hydroxide and phosphoric acid which are to be reacted in the synthesis process of the hydroxyapatite. By changing the Ca/P molar ratio of the starting hydroxyapatite, the proportions of $\alpha$-tricalcium phosphate and tetracalcium phosphate to be finally produced can be adjusted to desired values.

After synthesis by a suitable method such as a wet process, the starting hydroxyapatite is preferably rendered in powder form by suitable means such as filtration, centrifugation or spray-drying. It is also preferred for the hydroxyapatite to be thoroughly dried to remove as much water as possibly by a suitable method such as precalcination at a temperature of about from 500 to 700°C before it is thermally decomposed in a subsequent calcining step.

The thus prepared hydroxyapatite is calcined under reduced pressure at a temperature of from about 1,150°C to 1,450°C. If the calcining temperature is less than about 1,150°C, the intended thermal decomposition reaction will not take place to a practically acceptable extent even if the pressure is decreased.

The reason why the hydroxyapatite is calcined under reduced pressure is that the calcinating

3

temperature can be lowered so as to simplify the manufacturing steps and to reduce the production costs, and that a product of high activity can be attained. The reduced pressure under which the hydroxyapatite is calcined is preferably about 10 Pa or less, more preferably about $10^{-2}$ Pa or less.

The mixture of $\alpha$-tricalcium phosphate and tetracalcium phosphate which is prepared by thermal decomposition under reduced pressure of the hydroxyapatite having a Ca/P molar ratio of more than 1.5 but not exceeding about 1.8 can be used as a powder component of the composition of the present invention and in the process for producing a hardening material. Additionally, this mixture may be used as a powder for thermal spray as described for example, in Kinzoku Hyomen Gijuttsu Binran (Handbook of Metal Surface Treating Technology), pp. 1,132 to 1,139 (Nikkan Kogyo Shinbunsha, 1963) and Japanese Patent Application (OPI) No. 65871/88. If desired, the powder of the mixture may be granulated and fired at a temperature not higher than the point where conversion to hydroxyapatite takes place, thereby producing granules of bone prosthetic filler. These granules have the advantage that they can be placed at will in any areas of any shape that need prosthetic treatments. Other applications of the mixture include use as a column packing similar to the case of the hydroxyapatite.

The aqueous acidic solution for use as a hardening solution in the present invention may contain various inorganic and organic acids dissolved therein. Examples thereof include inorganic acids such as phosphoric acid, and organic acids such as acetic acid, lactic acid, citric acid, malic acid, malonic acid, succinic acid, glutaric acid, tartaric acid and polyacrylic acid. In the present invention, these acids are used in aqueous solution having acid concentrations which are preferably about 25 wt% or more, more preferably from about 25 to 55 wt%. If the acid concentration of the aqueous acidic solution is less than about 25 wt%, the hardening material obtained by mixing it with the powder component will not exhibit the desired strength.

In accordance with the present invention, polysaccharides are added and dissolved in the various aqueous acidic solutions described above. Any polysaccharides can be used if they are soluble in the solution and will not harm body tissues. Examples thereof include chitosan and starch, with chitosan being particularly preferred. The polysaccharides are dissolved in the aqueous acidic solutions in concentrations preferably about 0.05 wt% or more. The effectiveness of the polysaccharides is not sufficient if they are added in concentrations of less than about 0.05wt%.

By using the polysaccharide-containing aqueous acidic solutions as hardening solutions, the $\alpha$-tricalcium phosphate and/or tetracalcium phosphate as the powder component can be hardened mildly in the neutral range, and the composition for forming hardening materials is plastic enough to be readily formed into any complex shape.

The hardening reaction can be allowed to proceed even more mildly by further adding and dissolving at least one of glycerin, a monosaccharide, an oligosaccharide and a sugaralcohol in the aqueous acidic solution as the hardening solution having a polysaccharide dissolved therein. Examples of the monosaccharide include glucose and fructose, which may be used either alone or in admixture. Examples of the oligosaccharide include saccharose, maltose, lactose and raffinose, which may be used either alone or in admixture. Examples of the sugaralcohol include sorbitol, mannitol and xylitol, which may also be used either alone or in combination. Glycerin, monosaccharides, oligasaccharides and sugaralcohols may be used either independently or in combination.

When one or more of glycerin, monosaccharides, oligosaccharides and sugaralcohols are used, the total concentration thereof is preferably about 40 wt% or less, more preferably from about 5 to 40 wt%, and most preferably 10 to 30 wt.%. If the concentration of these additives exceeds about 40 wt%, increased difficulty will be encountered in dissolving them in the aqueous acidic solution.

In accordance with the process of the present invention, the powder component and the hardening solution prepared by the procedures described above are mixed so as to initiate the hydrolysis reaction of $\alpha$-tricalcium phosphate and/or tetracalcium phosphate, thereby producing hydroxyapatite to provide a hardening material. The powder component and the hardening solution are preferably mixed in such proportions that the ratio of the powder to the solution is within the range of from about 0.4 to 2.7 by weight more preferably from about 0.4 to 2.0 by weight. If the ratio of the powder to the solution is less than about 0.4, the solids content is too low to ensure desired strength for the hardened material. If the ratio of the powder to the solution exceeds about 2.7, it becomes difficult to attain uniform mixing of the powder and the solution.

The following examples are provided for the purpose of further illustration the present invention. All parts, ratio and presents are by weight unless otherwise indicated.

EXAMPLE 1

4

As aqueous solution of phosphoric acid was reacted with a suspension of calcium hydroxide by a conventional method. The reaction product was dried and calcined at 1,200°C to obtain $\alpha$-tricalcium phosphate in powder form. Two grams of this powder was mixed with 1 g of a hardening solution prepared by dissolving 0.1 g of chitosan ("Flonac N" of Kyowa Yushi Kogyo K.K.) in 10 g of an aqueous solution of 40% citric acid. A plastic gum-like composition was obtained and this composition hardened in about 7 minutes to produce a hardening material having a high strength.

EXAMPLE 2

An aqueous solution of phosphoric acid was reacted with a suspension of calcium hydroxide by a conventional method. The reaction product was dried and calcined at 1,200°C to obtain $\alpha$-tricalcium phosphate in powder form. Two grams of this powder was mixed with 1 g of a hardening solution prepared by dissolving 0.1 g of chitosan and 3 g of saccharose in 10 g of an aqueous solution of 40% citric acid. A plastic gum-like composition was obtained and this composition hardened in about 10 minutes to produce a hardening material having a high strength.

EXAMPLE 3

Calcium pyrophosphate was reacted with calcium carbonate by a conventional method, and calcined to obtain tetracalcium phosphate in powder form. Two grams of this powder was mixed with 1 g of a hardening solution prepared by dissolving 0.1 g of chitosan and 3 g of saccharose in 10 g of an aqueous solution of 40% citric acid. A plastic gum-like composition was obtained and this composition hardened in about 5 minutes to produce a hardening material having a high strength.

EXAMPLE 4

$\alpha$-Tricalcium phosphate and tetracalcium phosphate were prepared separately by conventional methods and mixed at a weight ratio of 2/1 to make a mixed powder. Two grams of this mixed powder was mixed with 1 g of a hardening solution prepared by dissolving 0.1 g of chitosan in 10 g of an aqueous solution of 40% citric acid. A plastic gum-like composition was obtained and this composition hardened in about 5 minutes to produce a hardening material having a high strength.

EXAMPLE 5

Two grams of the mixed powder prepared in Example 4 was mixed with 1 g of a hardening solution prepared by dissolving 0.1 g of chitosan and 3 g of saccharose in 10 g of an aqueous solution of 40% citric acid. A plastic gum-like composition was obtained and this composition hardened in about 7 minutes to produce a hardening material having a high strength.

EXAMPLE 6 TO 17

Two grams of a powder (indicated in Table 1) was mixed with 1 g of a hardening solution (indicated in Table 1). Similar to the cases of Examples 1 to 5, plastic gum-like compositions were obtained and hardening materials having a high strength could be produced from the compositions. The periods time required for these compositions to harden are also shown in Table 1, in which $\alpha$-TCP and COP denote $\alpha$-tricalcium phosphate and tetracalcium phosphate, respectively.

<u>TABLE 1</u>

Composition of hardening solution

| Example | Powder composition | Aqueous acidic solution | | Polysaccharide | | Other additives | | Time to harden (min) |
|---|---|---|---|---|---|---|---|---|
| | | acid and its concentration | amount (g) | | amount (g) | | amount (g) | |
| 6 | α-TCP | 40% citric acid | 10 | chitosan | 0.1 | glycerin | 3 | 10 |
| 7 | COP | 40% citric acid | 10 | chitosan | 0.1 | sorbitol | 3 | 5 |
| 8 | 2:1 mixture of α-TCP + COP | 40% citric acid | 10 | chitosan | 0.1 | glucose | 3 | 7 |
| 9 | α-TCP | 40% malic acid | 10 | chitosan | 0.1 | - | - | 7 |
| 10 | α-TCP | 40% malic acid | 10 | chitosan | 0.1 | glucose | 3 | 10 |
| 11 | COP | 40% malic acid | 10 | chitosan | 0.1 | glycerin | 3 | 5 |
| 12 | 2:1 mixture of α-TCP + COP | 40% malic acid | 10 | chitosan | 0.1 | sorbitol | 3 | 7 |
| 13 | COP | 40% malic acid | 10 | chitosan | 0.1 | saccharose | 3 | 5 |
| 14 | α-TCP | 40% phosphoric acid | 10 | chitosan | 0.1 | saccharose +glycerin | 3+3 | 2 |
| 15 | COP | 25% polyacrylic acid | 10 | chitosan | 0.1 | - | - | 10 |
| 16 | α-TCP | 25% polyacrylic acid | 10 | chitosan | 0.1 | glycerin | 3 | 20 |
| 17 | 2:1 mixture of α-TCP + COP | 25% polyacrylic acid | 10 | chitosan | 0.1 | saccharose | 3 | 15 |

COMPARATIVE EXAMPLE 1

The same procedures as in Example 1 were repeated except that chitosan was not added to the hardening solution. The composition obtained was not plastic and had a deteriorated formability although the composition hardened in about 2 minutes.

EXAMPLE 18

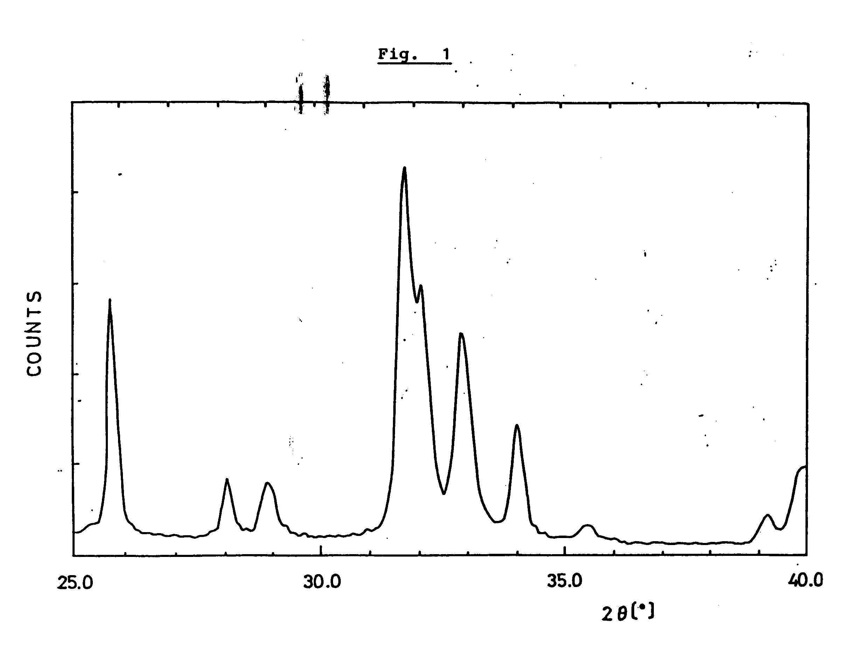

An aqueous solution of phosphoric acid was reacted with a suspension of calcium hydroxide by a conventional method, and the reaction product was dried to obtain hydroxyapatite. The identity of the product was established by X-ray diffraction, the results of which are shown by an X-ray diffraction scan in Fig. 1. A chemical analysis revealed that the resulting hydroxyapatite had a Ca/P molar ratio of 1.67.

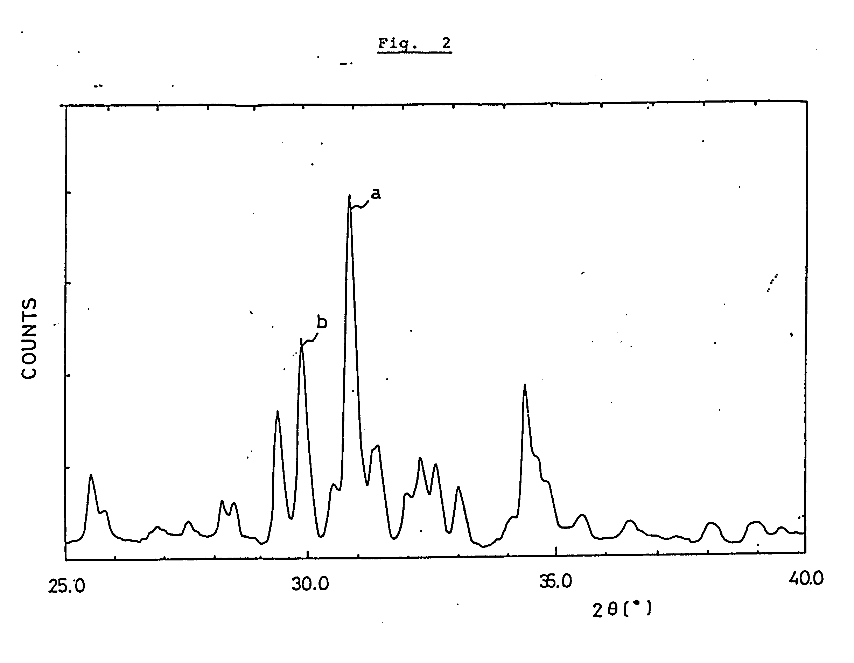

This hydroxyapatite was calcined at 1,200°C for 1 hour at a pressure of $1.3 \times 10^{-4}$ Pa. The resulting product was analyzed by X-ray diffraction as above and the results are shown in Fig. 2. The appearance of two characteristic peaks a ($\alpha$-tricalcium phosphate) and b (tetracalcium phosphate) verified the production of a mixture of $\alpha$-tricalcium phosphate and tetracalcium phosphate by thermal decomposition of hydroxyapatite.

Three grams of this mixture in powder form was mixed with 2 g of a commercial aqueous solution of gluconic acid (product of Wako Pure Chemical Industries, Ltd.; concentration, ca. 50%). The mixture underwent a hydrolytic reaction and hardened in about 1 hour.

EXAMPLE 19

The powder prepared as in Example 18 was mixed with hardening solutions (indicated in Table 2) at a weight ratio of 1/1 and hardening materials were obtained. The hardening times and the temperatures produced by the exothermic reaction of the hardening process are also shown in Table 2.

TABLE 2

| Composition of Hardening Solution (wt%) | | | | Hardening time (min) | Temperature (°C) |
|---|---|---|---|---|---|
| Citric acid | Glycerin | Sorbitol | Saccharose | | |
| 45 | 10 | 0 | 0 | 0.75 | 55 |
| 45 | 20 | 0 | 0 | 2 | 51 |
| 45 | 30 | 0 | 0 | 8 | 45 |
| 45 | 0 | 10 | 0 | 0.8 | 53 |
| 45 | 0 | 20 | 0 | 2 | 51 |
| 45 | 0 | 0 | 10 | 1 | 52 |
| 45 | 0 | 0 | 20 | 2.8 | 49 |
| 45 | 0 | 0 | 0 | 0.5 | 66 |

EXAMPLE 20

The mixture of $\alpha$-tricalcium phosphate and tetracalcium phosphate prepared in Example 18 was used as a powder component. Two grams of this powder was mixed with 1 g of a hardening solution prepared by dissolving 0.1 g of chitosan ("Flonac N" of Kyowa Yushi Kogyo K.K.) in 10 g of an aqueous solution of 40% citric acid. A plastic gum-like composition was obtained and this composition hardened in about 2 minutes to produce a hardening material having a high strength.

COMPARATIVE EXAMPLE 2

The same procedures as in Example 20 were repeated except that chitosan was not added to the hardening solution. The composition obtained was not plastic and had a deteriorated formability although the composition hardened in about 2 minutes.

EXAMPLE 21

The mixture of $\alpha$-tricalcium phosphate and tetracalcium phosphate prepared in Example 18 was used as a powder component. Two grams of this powder was mixed with 1 g of a hardening solution prepared by

EP 0 298 501 B1

dissolving 3 g of saccharose and 0.1 g of chitosan ("Flonac N" of Kyowa Yushi Kogyo K.K.) in 10 g of an aqueous solution of 40% citric acid. A plastic gum-like composition was obtained and this composition hardened in about 9 minutes to produce a hardening material having a high strength.

COMPARATIVE EXAMPLE 3

The same procedures as in Example 21 were repeated except that chitosan was not added to the hardening solution. The composition obtained was not plastic and had a deteriorated formability although the composition hardened in about 2 minutes.

In accordance with the present invention $\alpha$-tricalcium phosphate and/or tetracalcium phosphate is used as a powder and mixed with a hardening solution which is an aqueous acidic solution having a polysaccharide dissolved therein. The resulting composition is highly plastic and can be readily formed into a desired shape before hardening. The hardening reaction which occurs is mild and it proceeds at a moderate rate and uniformly to produce a hardening material having a high strength and which yet will not harm body tissues.

If a mixture of $\alpha$-tricalcium phosphate and tetracalcium phosphate is to be used as a powder component, it can be readily prepared by calcining a hydroxyapatite with a molar ratio of Ca/P of more than 1.5 and about 1.8 or less at a temperature of about from 1,150°C to 1,450 °C under reduced pressure. In this preferred embodiment, an aqueous acidic solution containing not only a polysaccharide but also at least one of glycerin, a monosaccharide, an oligosaccharide and a sugaralcohol may be used as a hardening solution. This offers further advantage of allowing the hardening reaction to proceed more uniformly to a fuller extent at a moderate and mild rate, thereby producing a hardening material having a high strength and which yet will not harm body tissues.

Therefore, the present invention provides a composition which is useful as a medical or dental cement material or a bone prosthetic material and which, immediately before use, is rendered sufficiently plastic by mixing the powder and the hardening solution to replace missing parts of a bone or tooth, and is subsequently hardened. In addition, the present invention provides a hardened biomaterial of a desired shape such as a hardening material in block form, an artificial bone or dental root.

**Claims**

1. A composition for forming a calcium phosphate type hardening material comprising (1) powder comprising at least one of $\alpha$-tricalcium phosphate and tetracalcium phosphate, (2) an acidic aqueous solution, and (3) a polysaccharide.

2. A composition according to claim 1, wherein said acidic aqueous solution contains at least one of glycerin, a monosaccharide, an oligosaccharide, and a sugar alcohol.

3. A composition according to claim 2, wherein the concentration of said at least one of glycerin, a monosaccharide, an oligosaccharide, and a sugaralcohol in said acidic aqueous solution is 40 wt% or less.

4. A composition according to claim 3, wherein the concentration of said at least one of glycerin, a monosaccharide, an oligosaccharide, and a sugaralcohol in said acidic aqueous solution is from 5 to 40 wt%.

5. A composition according to claim 4, wherein the concentration of said at least one of glycerin, a monosaccharide, an oligosaccharide, and a sugaralcohol in said acidic aqueous solution is from 10 to 30 wt%.

6. A composition according to any preceding claim, wherein said polysaccharide is dissolved in said acidic aqueous solution.

7. A composition according to claim 6, wherein the concentration of said polysaccharide in said aqueous solution is 0.05 wt% or more.

8. A composition according to any preceding claim, wherein said acidic aqueous solution is an aqueous solution of an organic acid.

8

EP 0 298 501 B1

9. A composition according to claim 8, wherein said organic acid is selected from acetic acid, lactic acid, citric acid, malic acid, malonic acid, succinic acid, glutaric acid, tartaric acid and polyacrylic acid.

10. A composition according to any preceding claim, wherein the acid concentration of said acidic aqueous solution is 25 wt% or more.

11. A composition according to claim 10, wherein the acid concentration of said acidic aqueous solution is from 25 to 55 wt%.

12. A composition according to claim 11, wherein said acidic aqueous solution is selected from a citric acid solution having an acid concentration of 40 wt%, a malic acid solution having an acid concentration of 40 wt%, and a polyacrylic acid solution having an acid concentration of 25 wt%.

13. A composition according to any of claims 1-7, wherein said acidic aqueous solution is an aqueous solution of an inorganic acid.

14. A composition according to claim 13, wherein said inorganic acid is phosphoric acid.

15. A composition according to claim 13 or claim 14, wherein the acid concentration of said acidic aqueous solution is 25 wt% or more.

16. A composition according to claim 15, wherein the acid concentration of said acidic aqueous solution is from 25 to 55 wt%.

17. A composition according to claim 16, wherein said acidic aqueous solution is a phosphoric acid solution having an acidic concentration of 40 wt%.

18. A composition according to any preceding claim, wherein said polysaccharide is chitosan.

19. A composition according to any preceding claim, wherein the ratio of said powder to said acidic aqueous solution is from 0.4 to 2.7 by weight.

20. A composition according to any preceding claim, wherein the ratio of said powder to said acidic aqueous solution is from 0.4 to 2.0 by weight.

21. A composition according to any preceding claim, wherein said powder is a mixture of $\alpha$-tricalcium phosphate and tetracalcium phosphate.

22. A composition as claimed in claim 21, wherein said powder is produced by the step of calcining, at a temperature of from 1,150°C to 1,450°C under reduced pressure, a hydroxyapatite having a molar ratio of Ca/P of 1.8 or less and more than 1.5.

23. A composition as claimed in claim 22, wherein said acidic aqueous solution is an aqueous solution of citric acid containing at least one of a monosaccharide, an oligosasccharide, and a sugar alcohol; and said polysaccharide is chitosan.

24. A composition according to claim 7, wherein said hydroxyapatite has a molar ratio of Ca/P of from 1.6 to 1.8.

25. A composition according to any of claims 22-24, wherein said hydroxyapatite is calcined under a pressure of 10 Pa or less.

26. A composition according to claim 25, wherein said hydroxyapatite is calcined under a pressure of $10^{-2}$ Pa or less.

27. A process for producing a calcium phosphate type hardening material comprising the step of mixing (1) powder comprising at least one of $\alpha$-tricalcium phosphate and tetracalcium phosphate and (2) an acidic aqueous solution containing a polysaccharide dissolved therein.

9

**Revendications**

1. Une composition pour former un matériau durcissable du type phosphate de calcium, comprenant (1) une poudre comprenant au moins l'un des phosphates α-tricalciques et tétracalciques, (2) une solution aqueuse acide, et (3) un polysaccharide.

2. Une composition selon la revendication 1, selon laquelle la solution aqueuse acide contient au moins l'un des suivants : la glycérine, un monosaccharide, un oligosaccharide et un alcool de sucre.

3. Une composition selon la revendication 2, selon laquelle la concentration de l'un au moins des suivants : la glycérine, un monosaccharide, un oligosaccharide et un alcool de sucre dans ladite solution aqueuse acide est de 40% en poids ou moins.

4. Une composition selon la revendication 3, selon laquelle la concentration de l'un au moins des suivants : la glycérine, un monosaccharide, un oligosaccharide, et un alcool de sucre dans ladite solution aqueuse acide est de 5 à 40% en poids.

5. Une compositions selon la revendication 4, selon laquelle la concentration de l'un au moins des suivants : la glycérine, un monosaccharide, un oligosaccharide et un alcool de sucre dans ladite solution aqueuse acide est de 10 à 30% en poids.

6. Une composition selon l'une quelconque des revendications précédentes, selon laquelle ledit polysaccharide est dissous dans ladite solution aqueuse acide.

7. Une composition selon la revendication 6, selon laquelle la concentration dudit polysaccharide dans ladite solution aqueuse est de 0,05% en poids ou plus.

8. Une composition selon l'une quelconque des revendications précédentes, selon laquelle ladite solution aqueuse acide est une solution aqueuse d'un acide organique.

9. Une composition selon la revendication 8, selon laquelle ledit acide organique est choisi parmi les suivants : acide acétique, acide lactique, acide citrique, acide malique, acide malonique, acide succinique, acide glutarique, acide tartrique et acide polyacrylique.

10. Une composition selon l'une quelconque des revendications précédentes selon laquelle la concentration d'acide de ladite solution aqueuse acide est de 25% en poids ou plus.

11. Une composition selon la revendication 10, selon laquelle la concentration d'acide de ladite solution aqueuse acide est de 25 à 55% en poids.

12. Une composition selon la revendication 11, selon laquelle ladite solution aqueuse acide est choisie parmi une solution d'acide citrique ayant une concentration d'acide de 40% en poids, une solution d'acide malique ayant une concentration d'acide de 40% en poids, et une solution d'acide polyacrylique ayant une concentration d'acide de 25% en poids.

13. Une composition selon l'une quelconque des revendications 1-7, selon laquelle ladite solution aqueuse acide est une solution aqueuse d'un acide inorganique.

14. Une composition selon la revendication 13, selon laquelle ledit acide inorganique est l'acide phosphorique.

15. Une composition selon la revendication 13 ou 14, selon laquelle la concentration d'acide de ladite solution aqueuse acide est de 25% en poids ou plus.

16. Une composition selon la revendication 15, selon laquelle la concentration d'acide de ladite solution aqueuse acide est de 25 à 55% en poids.

17. Une composition selon la revendication 16, selon laquelle ladite solution aqueuse acide est une solution d'acide phosphorique ayant une concentration d'acide de 40% en poids.

18. Une composition selon l'une quelconque des revendications précédentes, selon laquelle ledit polysaccharide est le chitosane.

19. Une composition selon l'une quelconque des revendications précédentes, selon laquelle le rapport de ladite poudre à ladite solution aqueuse acide est de 0,4 à 2,7 en poids.

20. Une composition selon l'une quelconque des revendications précédentes selon laquelle le rapport entre ladite poudre et ladite solution aqueuse acide est de 0,4 à 2,0 en poids.

21. Une composition selon l'une quelconque des revendications précédentes, selon laquelle ladite poudre est un mélange de phosphate α-tricalcique et de phosphate tétracalcique.

22. Une composition selon la revendication 21, selon laquelle ladite poudre est produite par l'étape de calcination, à une température de 1 150°C à 1 450°C sous pression réduite, d'une hydroxyapatite ayant un rapport molaire de Ca/P de 1,8 ou moins et supérieur à 1,5.

23. Une composition selon la revendication 22, selon laquelle ladite solution aqueuse acide est une solution aqueuse d'acide citrique contenant au moins l'un des suivants : un monosaccharide, un oligosaccharide et un alcool de sucre ; et ledit polysaccharide est le chitosane.

24. Une composition selon la revendication 7, selon laquelle ladite hydroxyapatite a un rapport molaire de Ca/P de 1,6 à 1,8.

25. Une composition selon l'une quelconque des revendications 22-24, selon laquelle ladite hydroxyapatite est calcinée sous une pression de 10 Pa ou moins.

26. Une composition selon la revendicaton 25, selon laquelle ladite hydroxyapatite est calcinée sous une pression de $10^{-2}$ Pa ou moins.

27. Un procédé de production d'un matériau durcissable du type phosphate de calcium comprenant l'étape de mélangeage de (1) une poudre comprenant au moins l'un des phosphates α-tricalciques et tétracalciques et (2) d'une solution aqueuse acide contenant un polysaccharide dissous.

**Patentansprüche**

1. Zusammensetzung zur Bildung eines Härtungsmaterials vom Kalziumphosphat-Typ, umfassend (1) Pulver, das zumindest eines von alpha-Trikalziumphosphat und Tetrakalziumphosphat enthält, (2) eine wässrige saure Lösung und (3) ein Polysaccharid.

2. Zusammensetzung nach Anspruch 1, dadurch **gekennzeichnet,** dass die saure wässrige Lösung zumindest eine Verbindung aus Glyzerin, einem Monosaccharid, einem Oligosaccharid und einem Zuckeralkohol enthält.

3. Zusammensetzung nach Anspruch 2, dadurch **gekennzeichnet,** dass die Konzentration der zumindest einen Verbindung aus Glyzerin, einem Monosaccharid, einem Oligosaccharid und einem Zuckeralkohol in der sauren wässrigen Lösung 40 Gew.% oder weniger ist.

4. Zusammensetzung nach Anspruch 3, dadurch **gekennzeichnet,** dass die Konzentration der zumindest einen Verbindung aus Glyzerin, einem Monosaccharid, einem Oligosaccharid und einem Zuckeralkohol in der sauren wässrigen Lösung von 5 bis 40 Gew.% ist.

5. Zusammensetzung nach Anspruch 4, dadurch **gekennzeichnet,** dass die Konzentration der zumindest einen Verbindung aus Glyzerin, einem Monosaccharid, einem Oligosaccharid und einem Zuckeralkohol in der sauren wässrigen Lösung von 10 bis 30 Gew.% ist.

6. Zusammensetzung nach einem der vorhergehenden Ansprüche, dadurch **gekennzeichnet,** dass das Polysaccharid in der sauren wässrigen Lösung aufgelöst ist.

7. Zusammensetzung nach Anspruch 6, dadurch **gekennzeichnet,** dass die Konzentration des Polysaccharids in der wässrigen Lösung 0,05 Gew.% oder mehr ist.

8. Zusammensetzung nach einem der vorhergehenden Ansprüche, dadurch **gekennzeichnet,** dass die saure wässrige Lösung eine wässrige Lösung einer organischen Säure ist.

9. Zusammensetzung nach Anspruch 8, dadurch **gekennzeichnet,** dass die organische Säure ausgewählt ist aus Essigsäure, Milchsäure, Zitronensäure, Äpfelsäure, Malonsäure, Succinsäure, Glutarsäure, Weinsäure und Polyacrylsäure.

10. Zusammensetzung nach einem der vorhergehenden Ansprüche, dadurch **gekennzeichnet,** dass die Säurekonzentration der sauren wässrigen Lösung 25 Gew.% oder mehr ist.

11. Zusammensetzung nach Anspruch 10, dadurch **gekennzeichnet,** dass die Säurekonzentration der sauren wässrigen Lösung 25 bis 55 Gew.% ist.

12. Zusammensetzung nach Anspruch 11, dadurch **gekennzeichnet,** dass die saure wässrige Lösung ausgewählt ist aus einer Zitronensäurelösung mit einer Säurekonzentration von 40 Gew.%, einer Äpfelsäurelösung mit einer Säurekonzentration von 40 Gew.% und einer Polyacrylsäurelösung mit einer Säurekonzentrtion von 25 Gew.%.

13. Zusammensetzung nach einem der Ansprüche 1 bis 7, dadurch **gekennzeichnet,** dass die saure wässrige Lösung eine wässrige Lösung einer anorganischen Säure ist.

14. Zusammensetzung nach Anspruch 13, dadurch **gekennzeichnet,** dass die anorganische Säure Phosphorsäure ist.

15. Zusammensetzung nach Anspruch 13 oder 14, dadurch **gekennzeichnet,** dass die Säurekonzentration der sauren wässrigen Lösung 25 Gew.% oder mehr ist.

16. Zusammensetzung nach Anspruch 15, dadurch **gekennzeichnet,** dass die Säurekonzentration der sauren wässrigen Lösung 25 bis 55 Gew.% ist.

17. Zusammensetzung nach Anspruch 16, dadurch **gekennzeichnet,** dass die saure wässrige Lösung eine Phosphorsäurelösung mit einer Säurekonzentration von 40 Gew.% ist.

18. Zusammensetzung nach einem der vorhergehenden Ansprüche, dadurch **gekennzeichnet,** dass das Polysaccharid Chitosan ist.

19. Zusammensetzung nach einem der vorhergehenden Ansprüche, dadurch **gekennzeichnet,** dass das Verhältnis des Pulvers zu der sauren wässrigen Lösung 0,4 bis 2,7 ist, bezogen auf das Gewicht.

20. Zusammensetzung nach einem der vorhergehenden Ansprüche, dadurch **gekennzeichnet,** dass das Verhältnis des Pulvers zu der sauren wässrigen Lösung 0,4 bis 2 ist, bezogen auf das Gewicht.

21. Zusammensetzung nach einem der vorhergehenden Ansprüche, dadurch **gekennzeichnet,** dass das Pulver eine Mischung aus alpha-Trikalziumphosphat und Tetrakalziumphosphat ist.

22. Zusammensetzung nach Anspruch 21, dadurch **gekennzeichnet,** dass das Pulver durch Kalzinieren eines Hydroxyapatits mit einem molaren Verhältnis von Ca/P von 1,8 oder weniger und mehr als 1,5 bei einer Temperatur von 1150°C bis 1450°C unter vermindertem Druck hergestellt ist.

23. Zusammensetzung nach Anspruch 22, dadurch **gekennzeichnet,** dass die saure wässrige Lösung eine Lösung aus Zitronensäure ist, die zumindest eine Verbindung aus einem Monosaccharid, einem Oligosaccharid und einem Zuckeralkohol enthält; und dass das Polysaccharid Chitosan ist.

**24.** Zusammensetzung nach Anspruch 7, dadurch **gekennzeichnet,** dass das Hydroxyapatit ein molares Verhältnis von Ca/P von 1,6 bis 1,8 aufweist.

**25.** Zusammensetzung nach einem der Ansprüche 22 bis 24, dadurch **gekennzeichnet,** dass das Hydroxyapatit bei einem Druck von 10 Pa oder weniger kalziniert ist.

**26.** Zusammensetzung nach Anspruch 25, dadurch **gekennzeichnet,** dass das Hydroxyapatit bei einem Druck von $10^{-2}$ Pa oder weniger kalziniert ist.

**27.** Verfahren zur Herstellung eines Härtungsmaterials vom Kalziumphosphat-Typ, umfassend das Mischen von (1) Pulver, das zumindest eine Verbindung von alpha-Trikalziumphosphat und Tetrakalziumphosphat enthält, und (2) einer sauren wässrigen Lösung, die ein darin aufgelöstes Polysaccharid enthält.

EP 0 298 501 B1

Fig. 1

14

Fig. 2

COUNTS

2θ(°)

25.0  30.0  35.0  40.0

EP 0 298 501 B1